Europäisches Patentamt
European Patent Office
Office européen des brevets

(11) Publication number: **0 367 410 A1**

# EUROPEAN PATENT APPLICATION

(21) Application number: 89310162.6

(22) Date of filing: 04.10.89

(51) Int. Cl.5: C07D 213/85, A01N 43/40, C07D 213/64, C07D 213/70

The title of the invention has been amended (Guidelines for Examination in the EPO, A-III, 7.3).

(30) Priority: 28.10.88 GB 8825314

(43) Date of publication of application: 09.05.90 Bulletin 90/19

(84) Designated Contracting States: AT BE CH DE ES FR GB GR IT LI LU NL SE

(71) Applicant: IMPERIAL CHEMICAL INDUSTRIES PLC
Imperial Chemical House, Millbank
London SW1P 3JF(GB)

(72) Inventor: Perrior, Trevor Robert
16 Silver Birches
Barkham Berks, RG11 4AD(GB)

(74) Representative: Ricks, Michael James et al
Imperial Chemical Industries PLC Legal Department: Patents PO Box 6 Bessemer Road
Welwyn Garden City Herts, AL7 1HD(GB)

(54) **Aryl pyridones as insecticidal agents.**

(57) Insecticidal compounds have the formula (I)

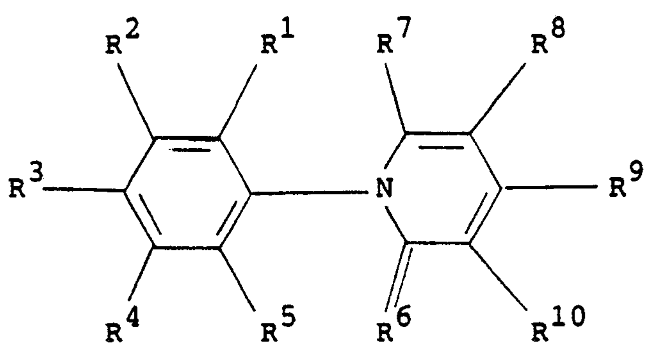

(I)

wherein $R^1$, $R^2$, $R^4$ and $R^5$ are independently selected from hydrogen, halogen, nitro, lower alkyl optionally substituted by halogen, lower alkoxy optionally substituted by halogen and lower alkenyl optionally substituted by halogen; $R^3$ is halogen, amino, mono- or di-(lower alkyl)-amino, lower alkyl substituted by halogen, lower alkoxy or lower alkenyl optionally substituted by halogen, provided that $R^3$ is not monochloro or monobromo- methyl; $R^6$ is oxygen or sulphur; $R^7$ is hydrogen, halogen, lower alkyl or lower alkoxy or lower thioalkoxy optionally substituted by halogen; $R^8$ is hydrogen, halogen, optionally substituted lower alkyl lower alkoxy or thioalkoxy, cyano, nitro, optionally substituted oximino, lower alkenyl, aryloxy, or amino or $S(O)nR^{11}$ wherein n is 0, 1 or 2 and $R^{11}$ is optionally substituted lower alkyl; $R^9$ is hydrogen, or lower alkyl or alkenyl optionally substituted by halogen, or $CO_2R^{12}$ wherein $R^{12}$ is lower alkyl optionally substituted by halogen; and is nitro or cyano.

EP 0 367 410 A1

## CHEMICAL COMPOUNDS

This invention relates to novel aryl pyridones useful as insecticidal agents.

The invention provides a compound of formula (I)

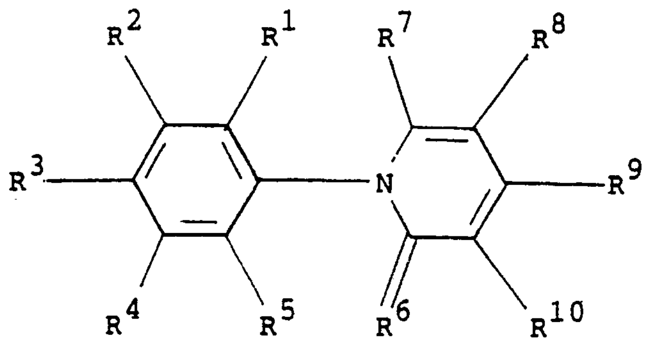

(I)

wherein $R^1$, $R^2$, $R^4$ and $R^5$ are independently selected from hydrogen, halogen, nitro, lower alkyl optionally substituted by halogen, lower alkoxy optionally substituted by halogen and lower alkenyl optionally substituted by halogen;

$R^3$ is halogen, amino, mono- or di-(lower alkyl)-amino, lower alkyl substituted by halogen, lower alkoxy optionally substituted by halogen and lower alkenyl optionally substituted by halogen provided that $R^3$ is not monochloro or monobromo- methyl;

$R^6$ is oxygen or sulphur;

$R^7$ is hydrogen, halogen, lower alkyl optionally substituted by halogen, lower alkoxy optionally substituted by halogen, and lower thioalkoxy optionally substituted by halogen; thioalkoxy optionally substituted by halogen; and

$R^8$ is hydrogen, halogen, optionally substituted lower alkyl, optionally substituted lower alkoxy, optionally substituted lower thioalkoxy, cyano, nitro, optionally substituted oximino, optionally substituted lower alkenyl, optionally substituted aryloxy, optionally substituted amino or $S(O)nR^{11}$ wherein n is 0, 1 or 2 and $R^{11}$ is optionally substituted lower alkyl;

$R^9$ is hydrogen, or lower alkyl optionally substituted by halogen, lower alkenyl optionally substituted by halogen or $CO_2R^{12}$ wherein $R^{12}$ is lower alkyl optionally substituted by halogen;

and $R^{10}$ is nitro or cyano.

As used herein the term "lower" used in relation to alkyl or alkoxy groups means groups having from 1 to 6 carbon atoms preferably from 1 to 3 carbon atoms and when used in relation to alkenyl groups means groups having from 2 to 6 carbon atoms, preferably 2 or 3 carbon atoms. The term "aryl" includes phenyl.

Suitably $R^1$, $R^2$, $R^3$, $R^4$ and $R^5$ are selected from hydrogen; halogen such as fluorine, chlorine or bromine; nitro; lower alkyl such as methyl or ethyl; lower alkoxy, such as methoxy; and halo(lower)alkyl such as trifluoromethyl and difluoromethyl.

Preferred examples of $R^1$ and $R^5$ include hydrogen, fluorine, chlorine, bromine and nitro.

Preferably both $R^1$ and $R^5$ are chlorine.

Preferred examples of $R^2$ and $R^4$ include hydrogen, halogen in particular, fluorine, lower alkyl such as methyl or ethyl and lower alkoxy such as methoxy.

Preferred examples of $R^3$ include halogen such as fluorine and chlorine, amino and halo lower alkyl such as trifluoromethyl and difluoromethyl.

Preferably $R^6$ is oxygen.

Examples of suitable halogen atoms for $R^7$ and $R^8$ include bromo.

Preferably $R^7$ is hydrogen.

Examples of $R^8$ include hydrogen, halo, lower alkyl optionally substituted by halo or hydroxy; cyano; nitro; oximino optionally substituted by lower alkyl, aryl, lower alkenyl or aralkyl wherein the aryl portion is optionally substituted with halogen or nitro; lower alkenyl optionally substituted by halogen or cyano; amino; or $S(O)R^{11}$ wherein n is 0, 1 or 2 and $R^{11}$ is lower alkyl optionally substituted by halogen such as fluorine.

Specific examples of $R^8$ include hydrogen, iodo, cyano methyl, hydroxymethyl, chloromethyl, difluoromethyl, dichloromethyl, trifluoromethyl, thiomethyl, ethoxyimino, t- butyloximino, p-nitrobenzyloxyimino, phenoxyimino, pentafluorobenzyl-oximino, prop-2-enyloxyimino, 2,2-dichloroethenyl, 2-cyanoethenyl,

ethynyl or $S(O)CF_3$.

Preferably $R^8$ is trifluoromethyl.

Suitable groups $R^9$ include hydrogen, halo(lower)alkyl, branched chain lower alkyl, halo(lower)alkenyl, or a lower carboxylic ester group.

Examples of suitable groups $R^9$ include hydrogen trifluoromethyl, 2,2-di-bromoethenyl, ethoxycarbonyl and tert- butyl.

Specific examples of compounds of formula I are set out in Table I.

TABLE I

| COMPOUND | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|
| 1 | Cl | H | $CF_3$ | H | Cl | O | H | $CF_3$ | H | CN |
| 2 | Cl | H | $CF_3$ | H | Cl | O | H | $CF_3$ | H | $NO_2$ |
| 3 | $NO_2$ | H | $CF_3$ | H | Cl | O | H | $CF_3$ | H | CN |
| 4 | $NO_2$ | H | $CF_3$ | H | Cl | O | H | $CF_3$ | H | $NO_2$ |
| 5 | F | H | $CF_3$ | H | Cl | O | H | $CF_3$ | H | CN |
| 6 | F | H | $CF_3$ | H | Cl | O | H | $CF_3$ | H | $NO_2$ |
| 7 | Cl | $CH_3$ | $CF_3$ | H | Cl | O | H | $CF_3$ | H | CN |
| 8 | Cl | $CH_3$ | $CF_3$ | H | Cl | O | H | $CF_3$ | H | $NO_2$ |
| 9 | Cl | H | $CHF_2$ | H | Cl | O | H | $CF_3$ | H | CN |
| 10 | Cl | H | $CHF_2$ | H | Cl | O | H | $CF_3$ | H | $NO_2$ |
| 11 | Cl | H | $CF_3$ | H | Cl | S | H | $CF_3$ | H | CN |
| 12 | Cl | H | $CF_3$ | H | Cl | S | H | $CF_3$ | H | $NO_2$ |

The compounds of formula I may be prepared by reacting a compound of formula (II):

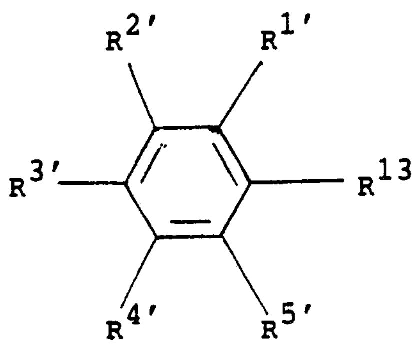

(II)

wherein $R^{1'}$, $R^{2'}$, $R^{3'}$, $R^{4'}$ and $R^{5'}$ are either $R^1$, $R^2$, $R^3$, $R^4$ and $R^5$ respectively as hereinbefore defined or are a precursor thereof and $R^{13}$ is a leaving group such as halo, with a compound of formula (III):

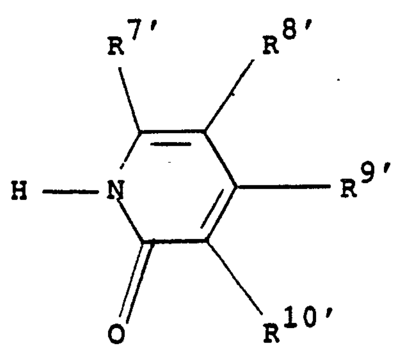

(III)

where $R^{7'}$, $R^{8'}$, $R^{9'}$ and $R^{10'}$ are either $R^7$, $R^8$, $R^9$ and $R^{10}$ respectively as hereinbefore defined or are a

precursor thereof and thereafter

(i) if any one or more of the groups $R^{1'}$, $R^{2'}$, $R^{3'}$, $R^{4'}$, $R^{5'}$, $R^{7'}$, $R^{8'}$, $R^{9'}$ and $R^{10'}$ is a precursor of the respective groups $R^1$, $R^2$, $R^3$, $R^4$, $R^5$, $R^7$, $R^8$, $R^9$ or $R^{10}$, converting the relevant precursor group $R^{1'}$, $R^{2'}$, $R^{3'}$, $R^{4'}$, $R^{5'}$, $R^{7'}$, $R^{8'}$, $R^{9'}$ or $R^{10'}$ to the group $R^1$, $R^2$, $R^3$, $R^4$, $R^5$, $R^7$, $R^8$, $R^9$ or $R^{10}$ respectively; or

(ii) if $R^6$ in formula (I) is sulphur, converting the carbonyl group to =S.

The reaction is suitably carried out in the presence of a solvent and a base. The base may be for example an alkali metal hydride, an alkali metal alkoxide or an alkali metal carbonate, and the solvent may be a hydrocarbon solvent, such as petroleum ether, an alcohol or an aprotic polar solvent such as dimethylformamide or dimethylacetamide. Suitable halo groups $R^{13}$ include fluoro, chloro, bromo or iodo and if necessary an appropriate catalyst such as a crown ether or copper can be added depending upon the precise nature of $R^{13}$. Further details of the processes for preparation of the compounds may be ascertained from the Examples set out hereinafter.

Optional step (ii) above may be carried out by reacting compounds of formula (I) wherein $R^6$ is oxygen with a thiolating agent such as phosphorus pentasulphide. The reaction is suitably carried out in an organic solvent such as pyridine at elevated temperatures of from 50 °C to 150 °C.

As used herein the term "precursor" applies to a group which can be converted to a chemical group $R^1$, $R^2$, $R^3$, $R^4$, $R^5$, $R^7$, $R^8$, $R^9$ or $R^{10}$ by standard chemical techniques. A particularly, useful precursor of this type is the formyl group as $R^{8'}$, $R^{9'}$ or $R^{10'}$. This group can be converted to various oxime, optionally substituted alkyl, optionally substituted alkenyl, optionally substituted alkynyl, and cyano groups as illustrated hereinafter.

For example compounds of formula (I) where $R^{10}$ is cyano can be prepared by reacting a compound of formula (IV)

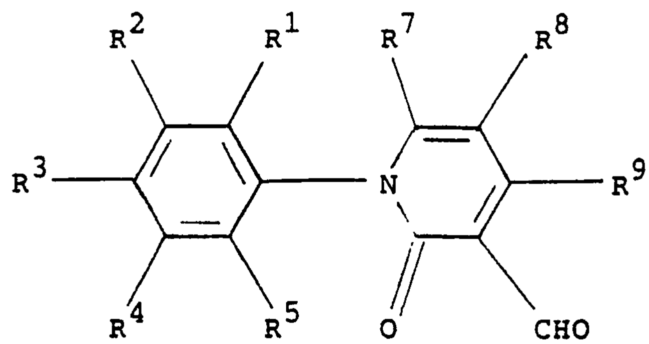

(IV)

wherein $R^1$, $R^2$, $R^3$, $R^4$, $R^5$, $R^7$, $R^8$ and $R^9$ are as hereinbefore defined with hydroxylamine hydrochloride in the presence of sodium formate and formic acid. An additional useful precursor of this type is the nitro group, in particular as $R^3$, as this can be converted to amino.,

A further useful precursor group, particularly for the $R^3$ group is ethoxycarbonyl. This group can be readily converted to various hydroxy alkyl groups such as hydroxy methyl or 1-methyl- 1-hydroxy ethyl by standard techniques such as by reduction or using Grignard reactions. The hydroxy alkyl groups can be converted directly to compounds of the invention, for example by halogenation or they may be converted to a second precursor such as formyl which is discussed above.

Compounds of formula (I) can be converted to other compounds of formula (I) having different substituent R groups by conventional methods if desired. For example, when one of $R^1$, $R^2$, $R^3$, $R^4$ or $R^5$ is fluoro, it can be converted to an alkoxy group by reaction with an appropriate alkoxy anion of formula $R^{14}O^-$ where $R^{14}$ is lower alkyl. Anions of formula $R^{14}O^-$ may be prepared by dissolving sodium metal in an alcohol of formula $R^{14}OH$ suitably at moderate temperatures of from 0 to 100 °C, preferably at room temperature.

Compounds of formula (II) are largely known compounds or they can be produced from known compounds by conventional methods. Compounds of formula (IV) can be prepared by oxidation of a compound of formula (VII)

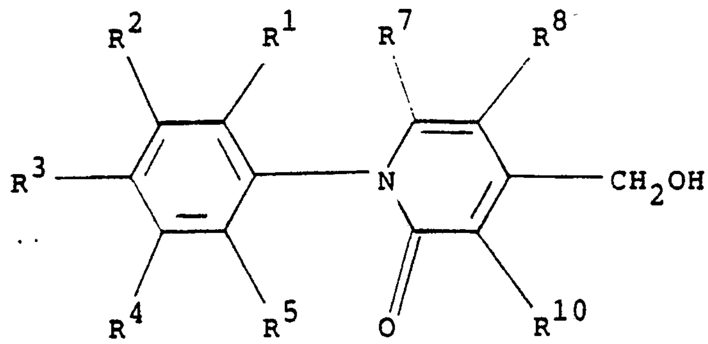

(VII)

wherein $R^1$, $R^2$, $R^3$, $R^4$, $R^5$, $R^7$, $R^8$ and $R^{10}$ are as hereinbefore defined. The oxidation is suitably carried out using an oxidising agent for example using Swern oxidation procedures. The reaction is suitably carried out in an organic solvent such as dichloromethane in the presence of a base low temperatures for example of from -100° C to 0° C are suitably employed.

Compounds of formula (VII) can be prepared by reduction of a compound of formula (I) wherein $R^9$ is $CO_2R^{12}$ using a reducing agent such as lithium borohydride. The reaction is suitably carried out in an inert organic solvent such as tetrahydrofuran at temperatures of from 0-150° C.

Compounds of formula (III) wherein $R^{10}$ is formyl can be prepared by reacting a compound of formula (VIII)

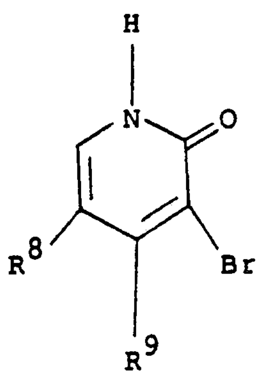

(VIII)

wherein $R^8$ and $R^9$ are as hereinbefore defined; with an electrophilic formylating agent, such as dimethylformamide in the presence of a base. Examples of such reactions are given hereinafter. The base used in the reaction is preferably a combination of sodium hydride followed by t-butyllithium. Suitable 3 equivalents of base are employed. The reaction is preferably carried out in an inert organic solvent such as tetrahydrofuran.

Other compounds of formula (III) and compounds of formula (VIII) are either known compounds or they can be produced from known compounds by conventional methods.

The compounds of formula (I) may be used to combat and control infestations of insect pests and also other invertebrate pests, for example, acarine pests. The insect and acarine pests which may be combated and controlled by the use of the invention compounds include those pests associated with agriculture (which term includes the growing of crops for food and fibre products), horticulture and animal husbandry), forestry, the storage of products of vegetable origin, such as fruit, grain and timber, and also those pests associated with the transmission of diseases of man and animals.

In order to apply the compounds to the locus of the pests they are usually formulated into compositions which include in addition to the insecticidally active ingredient or ingredients of formula I suitable inert diluent or carrier materials, and/or surface active agents. The compositions may also comprise another pesticidal material, for example another insecticide or acaricide, or a fungicide, or may also comprise an insecticide synergist, such as for example dodecyl imidazole, safroxan, or piperonyl butoxide.

The compositions may be in the form of dusting powders wherein the active ingredient is mixed with a solid diluent or carrier, for example kaolin, bentonite, kieselguhr, or talc, or they may be in the form of granules, wherein the active ingredient is absorbed in a porous granular material for example pumice.

Alternatively the compositions may be in the form of liquid preparations to be used as dips or sprays, which are generally aqueous dispersions or emulsions of the active ingredient in the presence of one or more known wetting agents, dispersing agents or emulsifying agents (surface active agents).

Wetting agents, dispersing agents and emulsifying agents may be of the cationic, anionic or non-ionic type. Suitable agents of the cationic type include, for example, quaternary ammonium compounds, for example cetyltrimethyl ammonium bromide. Suitable agents of the anionic type include, for example, soaps, salts of aliphatic monoesters of sulphuric acid, for example sodium lauryl sulphate, salts of sulphonated aromatic compounds, for example sodium dodecylbenzenesulphonate, sodium, calcium or ammonium lignosulphonate, or butylnaphthalene sulphonate, and a mixture of the sodium salts of diisopropyl- and triisopropylnaphthalene sulphonates. Suitable agents of the non-ionic type include, for example, the condensation products of ethylene oxide with fatty alcohols such as oleyl alcohol or cetyl alcohol, or with alkyl phenols such as octyl phenol, nonyl phenol and octyl cresol. Other non-ionic agents are the partial esters derived from long chain fatty acids and hexitol anhydrides, the condensation products of the said partial esters with ethylene oxide, and the lecithins.

The compositions may be prepared by dissolving the active ingredient in a suitable solvent, for example, a ketonic solvent such as diacetone alcohol, or an aromatic solvent such as trimethylbenzene and adding the mixture so obtained to water which may contain one or more known wetting, dispersing or emulsifying agents.

Other suitable organic solvents are dimethyl formamide, ethylene dichloride, isopropyl alcohol, propylene glycol and other glycols, diacetone alcohol, toluene, kerosene, white oil, methylnaphthalene, xylenes and trichloroethylene, N-methyl-2- pyrrolidone and tetrahydrofurfuryl alcohol (THFA).

The compositions which are to be used in the form of aqueous dispersions or emulsions are generally supplied in the form of a concentrate containing a high proportion of the active ingredient or ingredients, the said concentrate to be diluted with water before use. These concentrates are often required to withstand storage for prolonged periods and after such storage, to be capable of dilution with water to form aqueous preparations which remain homogeneous for a sufficient time to enable them to be applied by conventional spray equipment. The concentrates may contain 10-85% by weight of the active ingredient or ingredients. When diluted to form aqueous preparations such preparations may contain varying amounts of the active ingredient depending upon the purpose for which they are to be used. For agricultural or horticultural purposes, an aqueous preparation containing between 0.0001% and 0.1% by weight of the active ingredient (approximately equivalent to from 5-2000g/ha) is particularly useful.

In use the compositions are applied to the pests, to the locus of the pests, to the habitat of the pests, or to growing plants liable to infestation by the pests, by any of the known means of applying pesticidal compositions, for example, by dusting or spraying.

The compounds of the invention may be the sole active ingredient of the composition or they may be admixed with one or more additional active ingredients such as insecticides, insecticide synergists, herbicides, fungicides or plant growth regulators where appropriate. Suitable additional active ingredients for inclusion in admixture with the compounds of the invention may be compounds which will broaden the spectrum of activity of the compounds of the invention or increase their persistence in the location of the pest. They may synergise the activity of the compound of the invention or complement the activity for example by increasing the speed of effect, improving knockdown or overcoming repellency. Additionally multi-component mixtures of this type may help to overcome or prevent the development of resistance to individual components.

The particular insecticide, herbicide or fungicide included in the mixture will depend upon its intended utility and the type of complementary action required. Examples of suitable insecticides include the following:

a) Pyrethroids such as permethrin, esfenvalerate, deltamethrin, cyhalothrin in particular lambda-cyhalothrin, biphenthrin, fenpropathrin, cyfluthrin, tefluthrin, fish safe pyrethroids for example ethofenprox, natural pyrethrin, tetramethrin, s-bioallethrin, fenfluthrin, prallethrin and 5-benzyl-3-furylmethyl-(E)-(1R,3S)-2,2-dimethyl-3-(2-oxothiolan-3-ylidenemethyl) cyclopropane carboxylate;

b) Organophosphates such as profenofos, sulprofos, methyl parathion, azinphos-methyl, demeton-s-methyl, heptenophos, thiometon, fenamiphos, monocrotophos, profenophos, triazophos, methamidophos, dimethoate, phosphamidon, malathion, chloropyrifos, phosalone, fensulfothion, fonofos, phorate, phoxim, pyrimiphos-methyl, fenitrothion or diazionon;

c) Carbamates (including aryl carbamates) such as pirimicarb, cloethocarb, carbofuran, ethiofencarb, aldicarb, thiofurox, carbosulfan, beniocarb, fenobucarb, propoxur or oxamyl;

d) Benzoyl ureas such as triflumeron, or chlorofluazuron;

e) Organic tin compounds such as cyhexatin, fenbutatin oxide, azocyclotin;

f) Macrolides such as avermectins or milbemyins, for example such as avamectin, avermectin, and milbemycin;

g) Hormones such as pheromones;

h) Organochlorine compounds such as benzene hexachloride, DDT, chlordane or dieldrin.

i) Amidines, such as chlordimeform or amitraz.

In addition to the major chemical classes of insecticide listed above, other insecticides having particular targets may be employed in the mixture if appropriate for the intended utility of the mixture. For instance selective insecticides for particular crops, for example stemborer specific insecticides for use in rice such as cartap or buprofezin can be employed. Alternatively insecticides specific for particular insect species/stages for example ovo-larvicides such as chofentezine, flubenzimine, hexythiazox and tetradifon, moltilicides such as dicofol or propargite, acaricides such as bromopropylate, chlorobenzilate, or growth regulators such as hydramethylon, cyromazin, methoprene, chlorofluazuron and diflubenzuron may also be included in the compositions.

Examples of suitable insecticide synergists for use in the compositions include piperonyl butoxide, sesamex, and dodecyl imidazole.

Suitable herbicides, fungicides and plant-growth regulators for inclusion in the compositions will depend upon the intended target and the effect required. An example of a rice selective herbicides which can be included is propanil, an example of a plant growth regulator for use in cotton is "Pix", and examples of fungicides for use in rice include blasticides such as blasticidin-S.

The ratio of the compound of the invention to the other active ingredient in the composition will depend upon a number of factors including type of target, effect required from the mixture etc.

However in general, the additional active ingredient of the composition will be applied at about the rate as it is usually employed, or at a slightly lower rate if synergism occurs.

The compounds of formula 1 and compositions comprising them have shown themselves active against a variety of insect and other invertebrate pests. They are particularly useful in controlling public health pests such as flies and cockroaches. They may also be active against organophosphate and pyrethroid resistant strains of pests such as houseflies (Musca domestica). They may be effective in combating both susceptible and resistant strains of the pests in their adult, larval and intermediate stages of growth, and may be applied to the infested host animal by topical, oral or parenteral administration.

The following Examples illustrate various aspects of this invention. In the Preparations and Examples the products were usually identified and characterised by means of nuclear magnetic resonance spectroscopy and infra red spectroscopy. In each case where a product is specifically named its spectral characteristics are consistent with the assigned structure.

## EXAMPLE 1

This Example illustrates the preparation of compound 1 (Table I).

3-Bromo-5-trifluoromethyl-2-pyridone (1g, 4.13 mmol) was added portionwise to a well stirred suspension of sodium hydride (0.11 g) in tetrahydrofuran (20 ml) at room temperature in a nitrogen atmosphere. When the evolution of hydrogen was complete, the mixture was cooled to -78 °C and a solution of t-butyllithium in pentane (2.6 M,), 3.5 ml, 9.1 mmol) added dropwise, ensuring that the temperature did not exceed -65 °C. The reaction mixture was stirred for five minutes and then dimethylformamide (1 ml) was added dropwise, resulting in an exotherm to -50 °C. The mixture was allowed to warm to room temperature and then partitioned between ethyl acetate and 2 M hydrochloric acid. The organic layer was washed with brine, dried over magnesium sulphate and the solvent removed in vacuo to afford an off-white solid which was purified by column chromatography on silica gel with 5% methanol-dichloromethane as eluent to afford 3-formyl-5-trifluoromethyl-2-pyridone (700 mg).

NMR δ($d_6$-DMSO) 12.90 (1H,brs), 10.23 (1H,s), 8.12 (1H,s), 7.98 (1H,s)

This compound (700 mg) was added portionwise to a suspension of sodium hydride (95 mg) in dimethylformamide (10 ml). When the evolution of gas was complete 3,5-dichloro-4- fluorobenzotrifluoride (1.7 g) was added, and the reaction mixture heated at 90 °C for sixteen hours. Upon cooling the mixture was poured into water and extracted with ethyl acetate. The organic layer was washed with water, dried ($MgSO_4$) and evaporated in vacuo to afford a crude product which was chromatographed on silica gel with 30% diethyl ether-hexane eluent to give 1-(2,6-dichloro-4- trifluoromethylphenyl) 3-formyl-5-trifluoro-methyl-2-pyridone as white crystals.

NMR δ($CDCl_3$) 10.30 (1H,s), 8.32 (1H,s), 7.84 (2H,s), 7.76 (1H,s).

The compound (0.5 g, 1.24 mmol) was added to a mixture of hydroxylamine hydrochloride (95 mg), sodium formate (95 mg) and 98% formic acid (3 ml) and heated at reflux under nitrogen for four hours. The reaction mixture was cooled, poured into water and extracted with diethylether. The ethereal portion was well washed with aqueous sodium bicarbonate, then with brine, dried over ($MgSO_4$) and evaporated in vacuo to give an off white residue which was chromatographed on silica gel with 20% diethyl ether in hexane as eluent to give white crystals (400 mg). Recrystallisation from a mixture of ethyl acetate and hexane afforded the pure desired 3-cyano-1-(2,6-dichloro-4-trifluoromethylphenyl)-5-trifluoro-methyl-2-pyridone (compound 1, 200 mg).

NMR $\delta$ ($CDCl_3$) 8.13 (1H,d), 7.82 (2H,s), 7.74 (1H,d);

$\nu$max (Nujol) 2240 $cm^{-1}$, 1700 $cm^{-1}$

## EXAMPLE 2

A solution of 3-nitro-5-trifluoromethyl-2-pyridone (200 mg) in dimethylformamide (4 ml) was added to sodium hydride (26 mg) in a dry flask under a nitrogen atmosphere. The mixture was stirred for thirty minutes and then 3,5-dichloro-4-fluoro benzotrifluoroide (700 mg) in DMF (6 ml) was added and the reaction mixture heated at 85° C for sixteen hours, followed by heating at 110° C for twenty-four hours. The reaction mixture was poured into water, acidified with dilute hydrochloric acid and extracted with ethyl acetate. The organic layer was washed with brine, dried ($MgSO_4$) and concentrated in vacuo to afford a yellow oil which was chromatographed on silica gel with 6:1 petrol/diethyl ether as eluent. The resulting product was triturated with pentane to afford 1-(2,6-dichloro-4-trifluoromethylphenyl)-3-nitro-5-trifluoromethyl-2-pyridone (compound 2) as a pale yellow solid (48 mg) which showed:

Melting Point 152.3-153.5° C; $\delta$($CDCl_3$) 8.60 (1H,m), 7.81 (3H,m)

## EXAMPLE 3

This Example illustrate the preparation of 1-(2-chloro-4-trifluoromethyl-6-nitrophenyl)-3-cyano-5-trifluoromethyl-2-pyridone (Compound 3 of Table I).

3-Formyl-5-trifluoromethyl-2-pyridone, contaminated with 3-bromo-5-trifluoromethyl-2-pyridone was prepared using the general method described in Example 1. A portion of this product (3.45g) was dissolved in a mixture of formic acid (41 ml), sodium formate (1.36g, 0.02 mol) and hydroxylamine hydrochloride (1.39g, 0.02 mol). The reaction mixture was stirred at ambient temperature for two hours and then heated at reflux for twenty hours. The reaction was allowed to cool and left to stand for seven days, and then poured into water and extracted with ethyl acetate. The organic layer was washed with saturated sodium bicarbonate and brine and then dried over magnesium sulphate. Evaporation under reduced pressure gave a 74:26 mixture of 3-cyano-5-trifluoromethyl-2-pyridone and 3-bromo-5-trifluoromethyl-2-pyridone (2.29g) (ratio assessed by glc analysis of trimethylsilylated sample).

The mixture was characterised by its IR spectrum as follows: $\nu$max 2210, 1705 $cm^{-1}$.

A solution of the 74:26 mixture of 3-cyano-5-trifluoromethyl-2-pyridone and 3-bromo-5-trifluoromethyl-2-pyridone (190mg, 0.95 mmole) in dry dimethylformamide (2ml) was added dropwise to a stirred suspension of pentane-washed sodium hydride (50mg of a 55% dispersion in oil, 1.1 mmol) in dry dimethylformamide (1ml), under nitrogen. Rapid evolution of hydrogen was observed. The mixture was cooled to 5° C and a solution of 3-chloro-4-fluoro-5-nitrobenzotrifluoride (370mg, 1.5 mmol) in dry dimethylformamide (1 ml) was added. The mixture darkened and after 15 minutes was allowed to warm to 20° C. After minutes, water (20 ml) was added and the resulting mixture extracted with ether (3 x 30ml). The combined ethereal extracts were washed with water (10 ml), dried over magnesium sulphate, filtered and the ether was evaporated. The remaining mixture was chromatographed on silica gel, eluting with chloroform. The major product was isolated as yellow crystals, washed with pentane and air-dried to yield 252 mg of the desired product of melting point 200-201° C.

NMR ($CDCl_3$) 7.8(1H, t), 8.1 (1H, d), 8.2(H, d), 8.4 (1H, d).

1-(2-chloro-4-trifluoromethyl-6-nitrophenyl)-3-bromo-5-trifluoromethyl-2-pyridone was also isolated as a minor product (50 mg, m.p. 131° C).

EXAMPLE 4

This Example illustrates the preparation of 1-(2-chloro-4-trifluoromethyl-6-nitrophenyl)-3-nitro-5-trifluoromethyl-2-pyridone (Compound 4).

A solution of 3-nitro-5-trifluoromethyl-2-pyridone (340mg, 1.63 mmol) in dry dimethylformamide (3ml) was added dropwise to a stirred suspension of pentane-washed sodium hydride (80 mg of a 55% dispersion in oil, 1.8 mmole) in dry dimethylformamide (2ml), under nitrogen. Rapid evolution of hydrogen was observed. The mixture was cooled to 5°C and a solution of 3-chloro-4-fluoro-5-nitrobenzotrifluoride (600mg, 2.5 mmole) in dry dimethylformamide (2ml) added. After 15 minutes the mixture was allowed to warm to 20°C. After 1 hour, water (20 ml) was added and the resulting mixture extracted with ether (3 x 30ml). The combined ethereal extracts were washed with water (10 ml), dried over magnesium sulphate, filtered and the ether was evaporated. The remaining crude product was purified by chromatography on silica gel (50g), eluting with chloroform. The resulting oil crystallised under pentane. The yellow crystals were filtered off and air-dried to yield 576 mg of the desired product having a melting point of 139°C.

NMR δ($CDCl_3$) 7.9(1H, t), 8.2(1H, d), 8.5(1H, d), 8.6(1H, d).

BIOLOGICAL DATA

The insecticidal activity of compounds of formula (I) is set out in the following Table II as a grading of A, B or C where A indicates that 80-100% mortality was observed, B indicates that 50-79% mortality was observed and C indicates that 0-49% mortality was observed. The tests were conducted by spraying a suitable support medium (eg, leaves of a suitable food plant, or filter paper) with a solution of the compound under test and placing the pests thereon. Assessment of mortality was made 72 hours after spraying. In the test the compounds were used in the form of aqueous composition prepared by dissolving the compound in mixture of solvents consisting of 1 part by volume of acetone and 1 part by volume of ethanol and diluting the solution with water containing 1% by volume of a wetting agent (Synperonic "NX -Synperonic" is a Registered Trade Mark).

TABLE II

| COMPOUND | RATE OF APPLICATION ppm | NC | MD | BG | HV | SP | DB |
|---|---|---|---|---|---|---|---|
| 1 | 500 | C | A | A | C | C | C |
| 2 | 500 | B | A | A | C | C | C |
| 3 | 500 | C | B | C | C | B | C |
| 4 | 500 | B | C | C | C | C | C |

TABLE III

| CODE LETTERS | TEST SPECIES | SUPPORT MEDIUM/FOOD | TYPE OF TEST | DURATION |
|---|---|---|---|---|
| · (TABLE III) | | | | (days) |
| NL | Nephotetlix cinticeps (green leaf hopper-nymphs) | Rice Plant | CT | 2 |
| SP | Spodoptera exigua (lesser army worm larvae) | Cotton Leaf | RT | 2 |
| DB | Diabrotica balteata (rootworm larvae) | Filter paper/maiz-e seed | RT | 3 |
| BG | Blattella germanica (cockroach nymphs) | Plastic pot/calf weenes | RT | 3 |
| MD | Musca domestica (houseflies - adults) | Cotton wool/sugar | CT | 1 |
| HV | Heliothis virescens | Cotton leaf | RT | 3 |

## Claims

1. The invention provides a compound of formula (I)

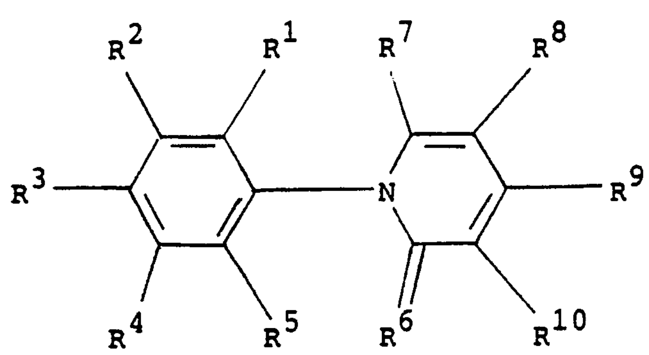

(I)

wherein $R^1$, $R^2$, $R^4$ and $R^5$ are independently selected from hydrogen, halogen, nitro, lower alkyl optionally substituted by halogen, lower alkoxy optionally substituted by halogen and lower alkenyl optionally substituted by halogen;
$R^3$ is halogen, amino, mono- or di-(lower alkyl)-amino, lower alkyl substituted by halogen, lower alkoxy optionally substituted by halogen and lower alkenyl optionally substituted by halogen provided that $R^3$ is not monochloro or monobromomethyl;
$R^6$ is oxygen or sulphur;
$R^7$ is hydrogen, halogen, lower alkyl optionally substituted by halogen, lower alkoxy optionally substituted by halogen, and lower thioalkoxy optionally substituted by halogen; thioalkoxy optionally substituted by halogen; and
$R^8$ is hydrogen, halogen, optionally substituted lower alkyl, optionally substituted lower alkoxy, optionally substituted lower thioalkoxy, cyano, nitro, optionally substituted oximino, optionally substituted lower alkenyl, optionally substituted aryloxy, optionally substituted amino or $S(O)nR^{11}$ wherein n is 0, 1 or 2 and $R^{11}$ is optionally substituted lower alkyl;
$R^9$ is hydrogen, or lower alkyl optionally substituted by halogen, lower alkenyl optionally substituted by

halogen or $CO_2R^{12}$ wherein $R^{12}$ is lower alkyl optionally substituted by halogen; and $R^{10}$ is nitro or cyano.

2. A compound according to claim 1 wherein $R^1$ and $R^5$ are independently selected from hydrogen, fluorine, chlorine, bromine or nitro.

3 A compound according to claim 2 wherein $R^1$ and $R^5$ are both chlorine.

4. A compound according to any of the preceding claims wherein $R^2$ and $R^4$ are independently selected from hydrogen, fluorine, methyl, ethyl or methoxy.

5. A compound according to any of the preceding claims wherein $R^3$ is fluorine, chlorine, amino, trifluoromethyl or difluoromethyl.

6. A compound according to any of the preceding claims wherein $R^6$ is oxygen.

7. A compound according to any of the preceding claims wherein $R^7$ is hydrogen and $R^8$ is trifluoromethyl.

8. A compound according to any of the preceding claims wherein $R^9$ is hydrogen, trifluoromethyl, 2,2-dibromoethenyl, ethoxycarbonyl or tert-butyl.

9. A method of killing or controlling insect or acarine pests which method comprises applying to the pest or to a locus thereof an insecticidally or acaricidally effective amount of a compound of formula (I) defined in claim 1.

10. An insecticidal or acaricidal composition comprising a compound of formula (I) as defined in claim 1 in combination with a diluent or carrier.

11. A method of preparing a compound of formula 1 as defined in claim 1 which comprises reacting a compound of formula (II):

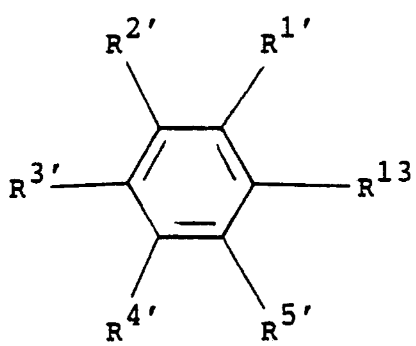

(II)

wherein $R^{1'}$, $R^{2'}$, $R^{3'}$, $R^{4'}$ and $R^{5'}$ are either $R^1$, $R^2$, $R^3$, $R^4$ and $R^5$ respectively as defined in claim 1 or are a precursor thereof and $R^{13}$ is a leaving group, with a compound of formula (III):

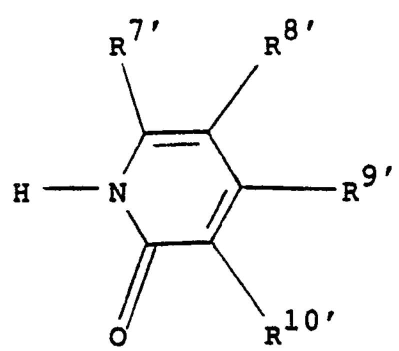

(III)

where $R^{7'}$, $R^{8'}$, $R^{9'}$ and $R^{10'}$ are either $R^7$, $R^8$, $R^9$ and $R^{10}$ respectively as defined in claim 1 or are a precursor thereof and thereafter,

(i) if any one or more of the groups $R^{1'}$, $R^{2'}$, $R^{3'}$, $R^{4'}$, $R^{5'}$, $R^{7'}$, $R^{8'}$, $R^{9'}$, $R^{10'}$ is a precursor of the respective group $R^1$, $R^2$, $R^3$, $R^4$, $R^5$, $R^7$, $R^8$, $R^9$ or $R^{10}$, converting the relevant precursor group, $R^{1'}$, $R^{2'}$, $R^{3'}$, $R^{4'}$, $R^{5'}$, $R^{7'}$, $R^{8'}$, $R^{9'}$ or $R^{10'}$ to the group $R^1$, $R^2$, $R^3$, $R^4$, $R^5$, $R^7$, $R^8$, $R^9$ or $R^{10}$ respectively; or

(ii) if $R^6$ in formula (I) is sulphur, converting the carbonyl group to =S.

European Patent Office

# EUROPEAN SEARCH REPORT

Application Number

EP 89 31 0162

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-1 900 947 (ROHM AND HAAS CO.) * Table II, especially page 19, 2nd compound and page 20, last compound * | 1,2,4,5,6,9 | C 07 D 213/85<br>A 01 N 43/40<br>C 07 D 213/64<br>C 07 D 213/70 |
| A | EP-A-0 259 048 (IMPERIAL CHEMICAL INDUSTRIES) * Whole document * | 1,9,11 | |
| A | EP-A-0 216 541 (IMPERIAL CHEMICAL INDUSTRIES) * Whole document * | 1,9,11 | |
| | | | TECHNICAL FIELDS SEARCHED (Int. Cl.5) |
| | | | C 07 D 213/00 |

The present search report has been drawn up for all claims

| Place of search | Date of completion of the search | Examiner |
|---|---|---|
| THE HAGUE | 15-01-1990 | DE JONG B.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)